# EUROPÄISCHE PATENTSCHRIFT

(11) **EP 1 070 741 B1**
(45) Veröffentlichungstag und Bekanntmachung des Hinweises auf die Patenterteilung: **09.04.2008**
(21) Anmeldenummer: 99114124.3
(22) Anmeldetag: 19.07.1999
(51) Int. Cl.: C08L 1/02, A61K 9/20, A61K 47/38, A61K 47/32

(54) **Coprozessiertes Polysaccharidprodukt mit vernetztem Polyvinylpyrrolidon**
Coprocessed polysaccharide product with crosslinked polyvinylpyrrolidone
Produit polysaccharidique co-travaillé avec du polyvinylpyrrolidone réticulé

(43) Veröffentlichungstag der Anmeldung: 24.01.2001
(73) Patentinhaber: fit GmbH, 02788 Hirschfelde (DE)
(72) Erfinder: Bauer, Kurt H., Prof. Dr., 79112 Freiburg (DE)
(74) Vertreter: Sommer, Peter

(56) Entgegenhaltungen:
- EP-A- 0 123 668
- EP-A- 0 690 302
- WO-A-98/03064
- US-A- 5 840 769

## Beschreibung

Die Erfindung betrifft mit vernetztem Polyvinylpyrrolidon als Supersprengmittel coprozessierte Celluloseprodukte, die sich einerseits durch leichte plastische Verformbarkeit, verbunden mit einer guten Tablettierbarkeit, und andererseits durch eine außerordentlich schnelle und starke Quellung beim Einbringen in Wasser oder wasserhaltige Flüssigkeiten auszeichnen. Dieser rasche Zerfall wird durch die innige Vermischung und Coprozessierung eines Supersprengmittels mit Cellulosepulver erreicht, wobei das Cellulosepulver wie ein Docht die Feuchtigkeit aufsaugt und dadurch das Supersprengmittel mit der zum optimalen Quellen und Sprengen notwendigen und ausreichenden Wassermenge versorgt. Die schnelle und starke Quellung wird durch eine möglichst hohe bzw. optimale Verdichtung des Zweikomponentensystems bei einem trockenen oder feuchten Coprozeß noch erheblich verbessert. Die neuen Produkte sind auch durch eine sehr gute Tablettierbarkeit charakterisiert. Sie können daher mit Erfolg als Tablettensprengmittel (Zerfallsbeschleuniger, Zerfallshilfsmittel) eingesetzt werden.

Als Tablettensprengmittel (Zerfallsbeschleuniger, Zerfallhilfsmittel) werden Hilfsstoffe bezeichnet, die für den raschen Zerfall von Tabletten in Wasser oder Magensaft und für die Freisetzung der Pharmaka in fein dispergierter, gut resorbierbarer Form sorgen. Je nach Wirkungsmechanismus handelt es sich um Substanzen, die die Porosität der Komprimate erhöhen und ein großes Adsorptionsvermögen für Wasser besitzen. Beispiele für bekannte Tablettensprengmittel und Supersprengmittel sind Stärke, Carboxymethylstärke, Cellulosederivate, Alginsäuren, Dextrane und quervernetzte Polyvinylpyrrolidone sowie gasentwickelnde Substanzgemische, z.B. Natriumhydrogencarbonat und Zitronen- oder Weinsäure (Brausemischungen).

Die im Stand der Technik bekannten Tablettensprengmittel, vor allem die Supersprengmittel Carboxymethylstärke und vernetztes Polyvinylpyrrolidon, sind wie Stärke aber noch mit folgenden Nachteilen behaftet:

Stärkekörner allein sind elastisch und deshalb schlecht plastisch verformbar bzw. schlecht tablettierbar und deshalb mehr Zerfallsbeschleuniger. Celluloseprodukte sind dagegen mehr plastisch und deshalb gut tablettierbar. Die Cellulosen wirken auch als Dochtsubstanzen, die ausreichende Wassermengen mit ausreichender Geschwindigkeit ansaugen, damit die sogenannten Supersprengmittel ihre Fähigkeiten voll entfalten können. Cellulosetabletten nehmen große Wassermengen auf und quellen auch sehr gut, ohne jedoch dabei zu zerfallen. Mischungen aus diesen beiden Substanzklassen vereinigen dagegen die Vorteile der guten Tablettierbarkeit, der starken Quellung bzw. der schnellen Zerfallbarkeit.

Der Erfindung liegt die Aufgabe zugrunde, das bekannte stark quellende Supersprengmittel vernetztes Polyvinylpyrrolidon zu verbessern und insbesondere Tablettensprengmittelprodukte bereitzustellen, die beim Einbringen in Wasser sofort, also in Sekundenschnelle, quellen, zerfallen und sich dispergieren. Daneben sollen je nach dem Vermahlungsgrad instantartig gröbere, feine und feinste bis kolloidartige Dispersionen herstellbar sein. Weiterhin sollen erfindungsgemäß Dispersions- bzw. Suspensionsstabilisatoren bereitgestellt werden, die mit vorteil bei der Herstellung von Pharmazeutika, Kosmetika und Nahrungsmitteln, die sich sehr rasch dispergieren sollen, verwendet werden können.

Diese Aufgabe wird erfindungsgemäß durch ein Polysaccharidprodukt, bestehend aus vernetztem Polyvinylpyrrolidon und pulverisierter oder mikrokristalliner Cellulose (MCC), gelöst. Vernetztes Polyvinylpyrrolidon wird auch als "Crospovidone" bezeichnet. Für die Zwecke der Erfindung können alle beliebigen im Handel erhältlichen vernetzten Polyvinylpyrrolidonsorten verwendet werden. Geeignete vernetzte Polyvinylpyrrolidonprodukte werden beispielsweise in den Firmenprospekten dieser Produkte der Firmen BASF AG, D-67056 Ludwigshafen/Rhein und ISP International Specialty Products, Wayne, N.J. 07470-3688 USA beschrieben.

Bevorzugte vernetzte Polyvinylpyrrolidonprodukte sind die handelsüblichen hochmolekularen, unlöslichen Polyvinylpyrrolidonsorten.

Das erfindungsgemäße, durch Coprozessierung von vernetztem Polyvinylpyrrolidon mit pulverisierter oder mikrokristalliner Cellulose (MCC) erhältliche Polysaccharidprodukt ist durch folgende Kenndaten charakterisiert:

Bei den gemahlenen, coprozessierten Zubereitungen sollen die Schüttdichten der Produkte mit Cellulosepulver bei 0,120-0,600 g/ml liegen, vorzugsweise bei 0,250-0,500 g/ml. Bei den Coprodukten mit mikrokristalliner Cellulose (MCC) und vernetztem Polyvinylpyrrolidon liegen die Schüttdichten bei 0,300-0,750 g/ml, vorzugsweise bei 0,350-0,600 g/ml. Die Schüttdichte des erfindungsgemäßen Polysaccharid-Produkts ist vorzugsweise höher als die Schüttdichte des Ausgangspulvergemisches.

Insbesondere ist das Produkt durch einen Restfeuchtegehalt von 1,5 bis 15%, vorzugsweise von weniger als 10%, beispielsweise 4 bis 7%, charakterisiert.

Der Gehalt der einzelnen Komponenten kann innerhalb weiter Grenzen variieren, beispielsweise von 1 bis 60 Gew.-% vernetztes Polyvinylpyrrolidon und 40 bis 99 Gew.-% pulverisierter oder mikrokristalliner Cellulose. Bevorzugt wird ein Gehalt von 3 bis 60 Gew.-% vernetztes Polyvinylpyrrolidon und 40 bis 97 Gew.-% pulverisierter oder mikrokristalliner Cellulose. Ganz bevorzugt werden Mengen von 5 bis 30 Gew.-% vernetztes Polyvinylpyrrolidon und 70 bis 95 Gew.-% pulverisierter oder mikrokristalliner Cellulose.

Auch die Korngröße des erfindungsgemäßen Polysaccharidprodukts kann innerhalb weiter Grenzen variieren, wie beispielsweise von 10 bis 2000 µm, vorzugsweise 50 bis 1000 µm.

Das erfindungsgemäße Polysaccharidprodukt kann durch Coprozessierung bzw. gemeinsame Behandlung von vernetztem Polyvinylpyrrolidon mit pulverisierter oder mikrokristalliner Cellulose durch feuchte oder trockene Aggregierung unter Druck erhalten werden. Unter dem Begriff "Coprozessierung" wird hier eine trockene Kompaktierung/Aggregierung, z.B. zwischen gegenläufigen Kompaktierwalzen bei Drücken von 20-60 kN, vorzugsweise 30-50 kN, oder eine feuchte Kompaktierung/ Aggregierung nach Zusatz von Wasser, durch Kneten oder Pressen von feuchtplastischen Massen durch ein Sieb, eine Lochscheibe oder über einen Extruder und abschließendem Trocknen verstanden.

Durch die Erfindung wird weiterhin ein Verfahren zur Herstellung des oben beschriebenen Polysaccharidprodukts bereitgestellt, welches die Coprozessierung von vernetztem Polyvinylpyrrolidon mit mikrokristalliner oder pulverisierter Cellulose umfaßt.

Eine Alternative des erfindungsgemäßen Verfahrens ist durch folgende Stufen charakterisiert:
(a) Suspendieren von feuchter bzw. nicht getrockneter, nicht verhornter mikrokristalliner oder pulverisierter Cellulose in einem, vorzugsweise heißen, wäßrigen Medium zu einer homogenen Dispersion;
(b) Vermengen der in Stufe (a) erhaltenen Suspension oder pastenförmigen vorverdichteten Massen nach Abkühlen unter 40°C mit vernetztem Polyvinylpyrrolidon;
(c) gegebenenfalls erfolgendes Vortrocknen;
(d) Aggregieren unter Druck, insbesondere verdichtendes Passieren, vorzugsweise Pressen der in Stufe (b) bzw. (c) erhaltenen und vorverdichteten feuchtplastischen Masse durch ein Sieb, eine Lochscheibe oder einen Extruder;
(e) Trocknen der in Stufe (d) erhaltenen aggregierten Masse bei einer Temperatur von 20 bis 90°C; und
(f) Vermahlen bzw. Brechen des so erhaltenen Produkts auf eine mittlere Korngröße von 10 bis 2000 µm, vorzugsweise 10 bis 1000 *µ*m.

In der ersten Stufe dieses Verfahrens wird feuchte, nicht verhornte pulverförmige oder mikrokristalline Cellulose in einem wäßrigen Medium zu einer homogenen Dispersion suspendiert.

Als Cellulose-Ausgangsprodukt wird vorzugsweise ein nicht getrocknetes mikrokristallines Celluloseprodukt oder pulverisierte Cellulose verwendet. Die pulverförmige Cellulose wird beispielsweise in an sich bekannter Weise aus Zellstoff oder faseriger Rohcellulose hergestellt. Die mikrokristalline Cellulose wird aus Zellstoffpulpe durch Erhitzen mit einer 3- bis 5%-igen starken Mineralsäure, beispielsweise Salz- oder Schwefelsäure, hergestellt. Für die Zwecke der Erfindung besonders geeignete, nicht getrockneten Celluloseprodukte sind praktisch Zwischen- oder Vorprodukte der Herstellung von reiner Pulvercellulose oder mikrokristalliner Cellulose.

Die oben erwähnte Herstellung von mikrokristalliner Cellulose aus Zellstoff oder von Pulvercellulose wird beispielsweise von B. Philipp und H.H. Steege in "Wissenschaft und Fortschritt" 25 (3), 126-131 (1975) beschrieben.

Unter "nicht getrocknet" werden Cellulosevorprodukte verstanden, die aus dem Herstellungsgang, nach der Reinigung, aber vor der Endtrocknung gewonnen werden, z.B. bei der oben beschriebenen Behandlung von Zellstoffpulpe mit Mineralsäure nach Entfernen der Säure und der erforderlichen Reinigung der hierbei entstandenen mikrokristallinen Cellulose. Das Cellulose:Wasser-Verhältnis kann 1,0:0,15 Teile bis 1,0:30,0 Teile, vorzugsweise 1,0:1,0 Teile bis 1,0:10,0 Teile betragen. Bei einer Trocknung des Produkts zu einem Feuchtigkeitsgehalt von weniger als 1,5 Teile Wasser pro 10,0 Teile Cellulose kann es vorkommen, daß die Oberfläche der Cellulose zu trocken wird und daß das Produkt in unerwünschter Weise "verhornt". In solchen Fällen sind die Polysaccharidprodukte nicht mehr optimal für die oben angegebenen Zwecke geeignet, da verhornte Bereiche nur mehr stark eingeschränkt hydratisieren können. Andererseits führen zu große Flüssigkeits- bzw. Wassermengen dazu, daß die Produkte nach der Zugabe von vernetztem Polyvinylpyrrolidon nicht ausreichend feuchtplastisch werden, sondern zu flüssig sind, so daß eine Obergrenze von 30,0 Teile, vorzugsweise 25,0 Teile Wasser pro 1,0 Teil Cellulose bevorzugt wird. Außerdem ist das Trocknen von hohen Feuchtigkeitsanteilen nicht wirtschaftlich. Relativ hohe Mengen von Wasser sind zu einer ausreichenden Verdichtung erforderlich, aber zu hohe Mengen können entsprechend den in Stufe (b) verwendeten Mengen des vernetzten Polyvinylpyrrolidon, je nach Vermahlungsgrad dieses Produkts die Gemische dann zu feucht werden lassen, so daß sie nicht mehr feuchtplastisch verformbar bzw. aggregierbar sind und nur noch langwierig unter Schwierigkeiten getrocknet werden können.

Somit wird in der ersten Stufe dieses Verfahrens die oben beschriebene nicht verhornte Cellulose in einem wäßrigen, zur besseren Hydratisierung vorzugsweise warmen bis heißen Medium zu einer homogenen Dispersion suspendiert. Als wäßriges Medium kann beispielsweise Wasser, wie Leitungswasser, verwendet werden, das gegebenenfalls bis zum Sieden aufgeheizt werden kann. Die Temperatur beim Suspendierungsvorgang beträgt 40 bis 100°C, vorzugsweise 80 bis 100°C. Die Suspendierung kann in an sich bekannter Weise in einer geeigneten Vorrichtung, wie einem Schnellmischer oder Dispergator, verwendet werden. Geeignete Vorrichtungen sind im Handel erhältlich und werden beispielsweise in Bauer, Frömming, Führer: Pharmazeutische Technologie, G. Fischer Verlag, Stuttgart, Jena, 5. Auflage, 1997, Seite 135 beschrieben. Bei dieser Befeuchtung, d.h. bei diesem Suspensionsvorgang, kann gegebenenfalls schon eine Vorverdichtung erzielt werden, z.B. durch stärkere Hydratation bei Verwendung von heißem Wasser. Weiterhin kann durch Kneten, beispielsweise im Z-Kneter mit kräftigen Mischarmen, im Gegensatz zu einfach gerührten bzw. gemischten Massen eine stärkere Verdichtung oder Vorverdichtung erreicht werden. Die so erhaltene Dispersion hat beispielsweise einen Feststoffgehalt von 10 bis 90 Gew.-%, vorzugsweise 20 bis 60 Gew.-%.

In der nächsten Stufe (b) dieses Verfahrens wird die in Stufe (a) erhaltene Suspension nach Abkühlung unter 40°C mit vernetztem Polyvinylpyrrolidon vermengt. Wie bereits oben ausgeführt, können in dieser Stufe alle beliebigen im Handel erhältlichen vernetzten Polyvinylpyrrolidonsorten (Crospovidones) verwendet werden. Bevorzugte Beispiele für geeignete Produkte sind oben angegeben.

Die Menge des in dieser Stufe zugegebenen vernetzten Polyvinylpyrrolidons wird so bemessen, daß die als Endprodukt erhaltenen Polysaccharidprodukte 3,0 bis 60 Gew.-%, vorzugsweise 5 bis 40 Gew.-%, vernetztes Polyvinylpyrrolidon enthalten.

Die Vermengung der beiden Ausgangsprodukte kann vorzugsweise durch Verkneten geschehen. Geeignete Kneter werden beispielsweise in Bauer, Frömming, Führer: Pharmazeutische Technologie, G. Fischer Verlag, Stuttgart, Jena, 5. Auflage, 1997, Seite 134-135 beschrieben. Die Temperatur bei der Vermengung bzw.

Verknetung beträgt 50 bis 30°C, vorzugsweise 40 bis 20°C. Vorzugsweise erfolgt die Verknetung bei Raumtemperatur, damit das Stärkeprodukt nicht nachteilig beeinflußt wird. Eine Verknetung kann daneben auch grundsätzlich dann vorgenommen werden, um eine Verdichtung zu erzielen.

In der nächsten Stufe (c) dieses Verfahrens wird das in Stufe (b) erhaltene Gemisch gegebenenfalls zu einem Restfeuchtegehalt von 1,5 bis 15 Gew.-%, vorzugsweise 4 bis 7 Gew.-%, vorgetrocknet. Als Trockner können übliche Trocknungsvorrichtungen, wie Trockenschränke und Wirbelschichttrockner eingesetzt werden. Geeignete Trockner werden beispielsweise in Bauer, Frömming, Führer: Pharmazeutische Technologie, G. Fischer Verlag, Stuttgart, Jena, 5. Auflage, 1997, Seiten 123-130 beschrieben. Bevorzugt wird die Vortrocknung bei Temperaturen von 30 bis 70°C, insbesondere 50 bis 60°C durchgeführt.

In der nächsten Stufe (d) dieses Verfahrens wird das in Stufe (b) bzw. (c) erhaltene feuchtplastische, durch das Befeuchten bereits vorverdichtete Produkt durch ein Sieb geschlagen bzw. gepreßt und dadurch möglichst weitgehend verdichtet. Als Siebe kommen beispielsweise übliche Siebe, die beispielsweise in Bauer, Frömming, Führer: Pharmazeutische Technologie, G. Fischer Verlag, Stuttgart, Jena, 5. Auflage, 1997, Seite 108 (Siebe), Seite 308 (Extrudergranulierung) beschrieben werden, in Betracht. Geeignete Siebe haben eine Maschenweite von 0,5 mm bis 5 mm, vorzugsweise 1-2 mm. Im übrigen erfolgt das Durchpressen bzw. -schlagen des Produkts durch Siebe bzw. durch Extruder in üblicher Weise und bei üblichen Bedingungen, wie Normaltemperatur. Das Sieben erfolgt bei Normaldruck oder gegebenenfalls unter Anwendung von Überdruck.

In der nächsten Stufe (e) dieses erfindungsgemäßen Verfahrens wird die durch das Sieb geleitete Masse bei einer Temperatur von 20 bis 90°C, vorzugsweise 50 bis 80°C getrocknet. Die Trocknung erfolgt in der gleichen Weise wie die oben im Zusammenhang mit Stufe (c) beschriebene Vortrocknung. Auch hier können übliche Trockner, wie Trockenschränke und Wirbelschichttrockner, eingesetzt werden.

In der letzten Stufe (f) dieses Verfahrens wird schließlich das so erhaltene Produkt auf eine mittlere Korngröße von 10 bis 1000 µm, vorzugsweise 50 bis 500 µm, vermahlt. Für den Vermahlungsvorgang können alle beliebigen geeigneten Mühlen, wie beispielsweise Kugelmühlen und dergleichen, verwendet werden. Geeignete Vorrichtungen sind beispielsweise in Bauer, Frömming, Führer: Pharmazeutische Technologie, G. Fischer Verlag, Stuttgart, Jena, 5. Auflage, 1997, Seite 104-107 beschrieben.

Eine andere sehr wirtschaftliche Alternative des erfindungsgemäßen Verfahrens ist durch folgende Stufen charakterisiert:
(a) Vermischen der pulverförmigen Bestandteile vernetztes Polyvinylpyrrolidon und Cellulose;
(b) Sieben durch ein feines Sieb;
(c) Verdichten durch Kompaktieren oder Brikettieren mit Kompaktierwalzen oder einer Exzenterpresse bei Drücken von 20-60 kN, vorzugsweise 30-50 kN;
(d) Granulieren bzw. verkleinerndes Homogenisieren der nach (c) erhaltenen Briketts oder Schülpen mit Stachelwalzen oder einem ähnlichen Gerät oder Vermahlen mit einer geeigneten Mühle auf Korngrößen von 10-2000 *µ*m; und
(e) Vermahlen des so erhaltenen Produkts auf eine mittlere Korngröße von 10 bis 1000 µm.

Bei dieser Alternative entfallen die Herstellungsschritte der Suspendierung (a), des Vortrocknens (c), das Pressen durch Siebe oder Extruder (d) und der Endtrocknung (e) der ersten Alternative. Es verbleiben nur die Schritte Vermischen der beiden Komponenten (a), einfaches Sieben (b), zum Beispiel durch ein Sieb mit einer Maschenweite von 1,2 mm, und als Hauptschritt die Kompaktierung mit Druck- bzw. Kalanderwalzen oder Exzenterpressen (c), die nachfolgende Zerkleinerung der kompaktierten Schülpen oder Briketts mit Stachelwalzen (d) und das Vermahlen mit geeigneten Mühlen (e).

Die Qualität der Verdichtung hängt von den Kompaktierdrucken (20-60 kN, vorzugsweise 30-50 kN) und in zweiter Linie auch noch von den Restfeuchtigkeitsgehalten der Mischung (b) ab, die zwischen 3 und 40 Gew.-%, vorzugsweise zwischen 5 und 25 Gew.-% liegen kann. Endprodukte mit den höheren Feuchtigkeitsgehalten müssen evtl. nachgetrocknet werden, bis sie Restfeuchtigkeiten von 1,5-15 Gew.-%, vorzugsweise von 4-7 Gew.-% aufweisen. Die Trockenverdichtungs- bzw. Trockengranulierverfahren werden beispielsweise in Bauer, Frömming, Führer: Pharmazeutische Technologie, G. Fischer Verlag, Stuttgart, Jena, 5. Auflage, 1997, Seite 306, 308 und 310 beschrieben.

Die so hergestellten erfindungsgemäßen, mit vernetztem Polyvinylpyrrolidon coprozessierten Celluloseprodukte zeichnen sich durch eine außerordentlich schnelle und starke Quellung beim Einbringen in Wasser oder wasserhaltige Flüssigkeiten sowie durch eine sehr gute Tablettierbarkeit aus. Sie können daher als äußerst wirksame Tablettensprengmittel (Zerfallhilfsmittel bzw. Zerfallsbeschleuniger) eingesetzt werden.

Charakteristisch für die erfindungsgemäßen, hochwirksamen Sprengmittel ist der durch die innige Mischung und Coprozessierung des Supersprengmittels mit der Cellulose erreichte innige Kontakt dieser beiden Hilfsstoffe. Dieser enge Kontakt soll auch nach dem Einarbeiten von Wirkstoffen (z.B. Arzneistoffe o.a.) und anderen Komponenten in die Tablettenrezeptur erhalten bleiben. Denn dieser enge Kontakt ist für die optimale Sprengwirkung entscheidend.

Ein weiterer Vorteil dieser intensiv coprozessierten, kombinierten Zerfallsbeschleuniger ist es, daß auch mechanisch feste, stark komprimierte, weniger poröse Tabletten hergestellt werden können.

Unter Verwendung der erfindungsgemäßen Produkte können Dispersionstabletten hergestellt werden, die beim Einbringen in Wasser sofort, also in Sekundenschnelle, quellen, zerfallen und sich dispergieren. Je nach Vermahlungsgrad des erfindungsgemäßen Produkts lassen sich aus den damit hergestellten Tabletten instantartig gröbere, feine und feinste bis kolloidartige Dispersionen erzeugen. Neben der Verwendbarkeit als Tablettenzerfallsbeschleuniger ist natürlich ein Einsatz als Dispersions- oder Suspensionsstabilisator bei der direkten Herstellung von flüssigen und halbfesten Arzneizubereitungen wie Sirupen, Lotionen, Säften, Gelen, Hydrosolen und Hydrogelen, Salbenzubereitungen und Pasten denkbar und möglich. Die Einsatzmöglichkeiten sind überall möglich, wo schnelle und feinste Dispergierungen erreicht und stabilisiert werden sollen. Die Anwendung ist nicht auf Pharmazeutika und Kosmetika beschränkt. Es drängen sich auch Verwendungen bei nahrungsmitteltechnologischen und technischen Aufgabenstellungen auf. Beispielsweise soll hier die Stabilisierung von Speiseeis oder Softeis, die Verdickung und Dispergierung von Soßen genannt werden, sowie auch Instantzubereitungen, wie Kakao- und andere Mixgetränke, die aus pulver- oder granulatartigen Vorprodukten augenblicklich anrührbar oder dispergierbar sein sollen.

Die Erfindung wird in den Beispielen erläutert.

### 1. Herstellungsbeispiele

### Beispiel 1:

1000,0 kg feuchtes, nicht verhorntes Cellulosepulver mit einem Wassergehalt von ca. 20% wird möglichst bald nach der Herstellung durch Zugabe von 2500,0 kg Wasser resuspendiert. Die Suspension wird mit einem Schnellmischer oder Dispergator homogen dispergiert, und anschließend werden 160,0 kg handelsübliches vernetztes Polyvinylpyrrolidon (z.B. Kollidon^{®} CL) eingeknetet. Die feuchtplastische Masse wird durch einen Extruder mit 1 mm Maschenweite geschlagen und in einem geeigneten Trockner bei 60°C getrocknet. Das fertig coprozessierte Produkt hat einen Restfeuchtegehalt von 5-8 Gew.-% und mittlere Partikelgrößen von 0,25-0,8 mm.

### Beispiel 2:

2250,0 kg feuchter Kuchen mit einem Verhältnis mikrokristalline Cellulose:Wasser = 40:60 wird mit der gleichen Menge Wasser resuspendiert und homogenisiert. Anschließend werden 100,0 kg vernetztes Polyvinylpyrrolidon (z.B. Crospovidone) gleichmäßig eingeknetet, die feuchtplastische Masse durch ein 1,5 mm Sieb geschlagen und in einem Wirbelschichttrockner (Zulufttemperatur 100-120°C) bis zu einer Restfeuchte von ca. 5% getrocknet (Temperatur der Ausgangsluft 35-45°C).

### Beispiel 3:

2000,0 kg feuchter Kuchen aus mikrokristalliner Cellulose und Wasser im Verhältnis 50:50 wird mit der doppelten Menge Wasser angeschlämmt bzw. resuspendiert und 5 min lang mit dem Ultra-Turrax homogenisiert.

Anschließend wird die homogene Mischung in einen Planetenmischen gegeben, und bei laufendem Rührwerk wird portionsweise 500,0 kg vernetztes Polyvinylpyrrolidon (z.B. Kollidon^{®} CL bzw. Crospovidone C oder M) dazugeknetet und abschließend 10 min gemischt.

Die feuchtplastische Masse wird in dünnen Schichten und angemessenen Portionen entweder im Trockenschrank bei 50°C oder im Wirbelschicht-Trockner bei einer Zulufttemperatur von 80-90°C bis zur siebbaren Konsistenz vorgetrocknet. Dann wird sie durch ein 5mm-Sieb geschlagen und im Wirbelschicht-Trockner bei einer Zulufttemperatur von 80°C zu Ende getrocknet (Restfeuchte ca. 5%) .

Zuletzt wird die Masse durch ein 1,00 mm-Sieb geschlagen und in einer geeigneten Mühle auf eine mittlere Korngröße von 80 µm gemahlen.

### Beispiel 4:

- 2000,0 g: feuchtes, feingemahlenes Cellulosepulver (mittlere Partikelgröße 50 µm, Feuchtigkeitsgehalt ca. 50%) wird mit
- 1500,0 g: siedendem, deionisiertem Wasser in einem geeigneten Mischer homogen befeuchtet. Anschließend werden
- 100,0 g: vernetztes Polyvinylpyrrolidon (z.B. Polyplasdone^{®} XL) eingeknetet, so daß eine feuchtplastische Masse entstanden ist. Die feuchtplastische Masse wird mit möglichst hohem Druck durch einen Extruder mit einer

### Beispiel 5:

- 9,0 kg: feingemahlenes Cellulosepulver (mittlere Partikelgröße 40 µm) und
- 1,0 kg: vernetztes Polyvinylpyrrolidon (z.B. Polyplasdone^{®} XL-10) werden homogen gemischt und durch ein 1 mm-Sieb geschlagen. Anschließend wird diese Mischung mit Hilfe einer Kompaktierwalze bei Drucken von 30-45 kN brikettiert und durch ein 0,2 mm- oder 1 mm-Sieb geschlagen. Die Schüttdichte des so hergestellten Produkts liegt bei 0,400-0,450 g/ml.

### 2. Verwendungsbeispiel

Unter Verwendung der erfindungsgemäßen, mit vernetztem Polyvinylpyrrolidon coprozessierten gemahlenen Cellulose wurden Tabletten in folgender Weise hergestellt:

| | |
|---|---|
| ASS (Acetylsalicylsäure), mittlere Korngröße 0,2 mm | 1000,0 |
| Cellulose-Crospovidone Coprocessed | 100,0 |

werden gemischt und auf einer üblichen Tablettenpresse zu Tabletten mit 11 mm Durchmesser und 550 mg Bruttogewicht verpreßt.

Die im Verwendungsbeispiel hergestellten Tabletten wurden folgendem Zerfallstest in folgender Weise unterworfen:
a) einfaches Einlegen der Tabletten in ein Becherglas mit ca. 200-250 ml reines Wasser bei Raumtemperatur und Beobachtung des Zerfalls;
b) Einbringen jeweils einer Tablette, die in einem Drahtbügel eingeklemmt ist, in ein Becherglas mit 250 ml reinem Wasser bei Raumtemperatur und Bestimmung des Zeitraums bis zum vollständigen Zerfall.

Die erhaltenen Ergebnisse sind in folgender Tabelle zusammengestellt:

| | a) | b) |
|---|---|---|
| Tabl. mit 500 mg ASS + 50 mg Crospovidone coprozessiert nach Beispiel 1: | zerfällt sehr rasch | 10-15 s |
| Tabl. mit 500 mg ASS + 50 mg Crospovidone coprozessiert nach Beispiel 2: | " | 20-25 s |
| Tabl. mit 500 mg ASS + 50 mg Crospovidone coprozessiert nach Beispiel 5: | " | 16-17 s |
| Tabl. mit 500 mg ASS + 50 mg Weizenstärke: | quillt stark, aber zerfällt nicht | 35-45 s |

Aus der obigen Tabelle ergibt sich, daß die unter Verwendung der erfindungsgemäßen Produkte hergestellten Tabletten im Vergleich zu den Produkten mit herkömmlichen Sprengmitteln wesentlich verbesserte Zerfallseigenschaften haben.

## Patentansprüche

1. Polysaccharidprodukt erhältlich durch das Verfahren nach einem der Ansprüche 6 bis 9.

2. Polysaccharidprodukt nach Anspruch 1, **dadurch gekennzeichnet, daß** es eine Restfeuchte von weniger als 10% besitzt.

3. Polysaccharidprodukt nach Anspruch 1 oder 2, **dadurch gekennzeichnet, daß** es 3 bis 60 Gew.-% vernetztes Polyvinylpyrrolidon und 40 bis 97 Gew.-% mikrokristalline oder pulverisierte Cellulose enthält.

4. Polysaccharidprodukt nach einem der Ansprüche 1 bis 3, **dadurch gekennzeichnet, daß** es eine mittlere Korngröße 10 bis 1000 µm hat.

5. Polysaccharidprodukt nach einem der Ansprüche 1 bis 4, **dadurch gekennzeichnet, dass** es gegenüber dem Ausgangspulvergemisch eine höhere Schüttdichte hat.

6. Verfahren zur Herstellung eines Polysaccharidprodukts mit einer mittleren Korngröße von 10 bis 2000 µm, bestehend aus vernetztem Polyvinylpyrrolidon und mikrokristalliner oder pulverisierter Cellulose, umfassend die Coprozessierung von vernetztem Polyvinylpyrrolidon mit mikrokristalliner oder pulverisierter Cellulose durch trockene Aggregierung bei Drücken von 20 - 60 kN oder feuchte Aggregierung nach Zusatz von Wasser durch verdichtendes Passieren der feuchtplastischen Masse durch ein Sieb, eine Lochscheibe oder einen Extruder und anschließendes Trocknen unter Druck.

7. Verfahren nach Anspruch 6, **dadurch gekennzeichnet, daß** es die folgenden Stufen umfaßt:
(a) Suspendieren und gegebenenfalls Verdichten von feuchter bzw. nicht getrockneter, nicht verhornter mikrokristalliner oder pulverisierter Cellulose in einem wäßrigen Medium zu einer homogenen Dispersion;
(b) Vermengen der in Stufe (a) erhaltenen Suspension oder pastenförmigen, vorverdichteten Massen nach Abkühlen unter 40°C mit vernetztem Polyvinylpyrrolidon;
(c) gegebenenfalls erfolgendes Vortrocknen;
(d) Verdichtendes Passieren, vorzugsweise Pressen der in Stufe (b) bzw. (c) erhaltenen und vorverdichteten feuchtplastischen Masse durch ein Sieb, eine Lochscheibe oder einen Extruder,
(e) Trocknen der in Stufe (d) erhaltenen aggregierten Masse bei einer Temperatur von 20 bis 90°C; und
(f) Vermahlen des so erhaltenen Produkts auf eine mittlere Korngröße von 10 bis 2000 µm, vorzugsweise 10-1000 µm.

8. Verfahren nach Anspruch 7, **dadurch gekennzeichnet, daß** das Vermengen und Verdichten durch Verkneten erfolgt.

9. Verfahren nach Anspruch 6, **dadurch gekennzeichnet, daß** es die folgenden Stufen umfaßt:
(a) Vermischen der pulverförmigen Bestandteile vernetztes Polyvinylpyrrolidon und Cellulose;
(b) Sieben durch ein feines Sieb;
(c) Verdichten durch Kompaktieren oder Brikettieren mit Kompaktierwalzen oder einer Exzenterpresse bei Drücken von 20-60 kN, vorzugsweise 30-50 kN;
(d) Granulieren bzw. verkleinerndes Homogenisieren der nach (c) erhaltenen Briketts oder Schülpen mit Stachelwalzen oder einem ähnlichen Gerät oder Vermahlen mit einer geeigneten Mühle auf Korngrößen von 10-2000 µm; und
(e) Vermahlen des so erhaltenen Produkts auf eine mittlere Korngröße von 10 bis 1000 µm.

10. Verwendung des Polysaccharidprodukts nach den Ansprüchen 1 bis 5 als Dispersions- oder Suspensionsstabilisator bei der Herstellung von flüssigen und halbfesten Zubereitungen.

11. Verwendung des Polysaccharidprodukts nach den Ansprüchen 1 bis 5 als Tablettensprengmittel.

## Claims

1. Polysaccharide product obtainable by the process according to one of Claims 6 to 9.

2. Polysaccharide product according to Claim 1,
**characterized in that** it has a residual moisture content of less than 10%.

3. Polysaccharide product according to Claim 1 or 2, **characterized in that** it contains 3 to 60% by weight of crosslinked polyvinylpyrrolidone and 40 to 97% by weight of microcrystalline or pulverized cellulose.

4. Polysaccharide product according to one of Claims 1 to 3, **characterized in that** it has a mean particle size of 10 to 1000 µm.

5. Polysaccharide product according to one of Claims 1 to 4, **characterized in that** it has a higher bulk density than the starting powder mixture.

6. Process for producing a polysaccharide product having a mean particle size of 10 to 2000 µm, consisting of crosslinked polyvinylpyrrolidone and microcrystalline or pulverized cellulose, comprising coprocessing of crosslinked polyvinylpyrrolidone with microcrystalline or pulverized cellulose by dry aggregation at pressures of 20-60 kN or moist aggregation after addition of water by compressive conveying of the wet-plastic material through a screen, a perforated disc or an extruder and subsequent drying under pressure.

7. Process according to Claim 6, **characterized in that** it comprises the following stages:
(a) suspending and optionally compressing moist or undried, unhornified microcrystalline or pulverized cellulose in an aqueous medium to give a homogeneous dispersion;
(b) mixing the suspension or pasty precompressed materials obtained in stage (a), after cooling to below 40°C, with crosslinked polyvinylpyrrolidone;
(c) optional predrying;
(d) compressive conveying, preferably pressing, of the precompressed wet-plastic material obtained in stage (b) or (c) through a screen, a perforated disc or an extruder,
(e) drying the aggregated material obtained in stage (d) at a temperature of 20 to 90°C; and
(f) grinding the product thus obtained to a mean particle size of 10 to 2000 µm, preferably 10-1000 µm.

8. Process according to Claim 7, **characterized in that** the mixing and compression are effected by kneading.

9. Process according to Claim 6, **characterized in that** it comprises the following stages:
(a) mixing the pulverulent constituents crosslinked polyvinylpyrrolidone and cellulose;
(b) screening through a fine screen;
(c) compression by compacting or briquetting with compacting rolls or an eccentric press at pressures of 20-60 kN, preferably 30-50 kN;
(d) granulation or particle size-reducing homogenization of the briquettes or slugs obtained in (c) with spiked rolls or a similar unit or grinding with a suitable mill to particle sizes of 10-2000 µm; and
(e) grinding the product thus obtained to a mean particle size of 10 to 1000 µm.

10. Use of the polysaccharide product according to Claims 1 to 5 as a dispersion or suspension stabilizer in the production of liquid and semisolid formulations.

11. Use of the polysaccharide product according to Claims 1 to 5 as a tablet disintegrant.

## Revendications

1. Produit polysaccharidique obtenu par le procédé selon l'une quelconque des revendications 6 à 9.

2. Produit polysaccharidique selon la revendication 1, **caractérisé en ce qu'**il possède une humidité résiduelle inférieure à 10 %.

3. Produit polysaccharidique selon la revendication 1 ou 2, **caractérisé en ce qu'**il contient 3 à 60 % en poids de polyvinylpyrrolidone réticulée et 40 à 97 % en poids de cellulose microcristalline ou pulvérisée.

4. Produit polysaccharidique selon l'une quelconque des revendications 1 à 3, **caractérisé en ce qu'**il a une granulométrie moyenne de 10 à 1000 µm.

5. Produit polysaccharidique selon l'une quelconque des revendications 1 à 4, **caractérisé en ce qu'**il a une densité apparente supérieure à celle du mélange pulvérulent de départ.

6. Procédé pour préparer un produit polysaccharidique avec une granulométrie moyenne de 10 à 2000 µm, constitué par de la polyvinylpyrrolidone réticulée et de la cellulose microcristalline ou pulvérisée, comprenant le co-traitement de la polyvinylpyrrolidone réticulée avec la cellulose microcristalline ou la cellulose pulvérisée par une agrégation à l'état sec à des pressions de 20 à 60 kN ou par une agrégation à l'état humide après addition d'eau en faisant passer par compression la masse plastique humide à travers un crible, un disque perforé ou une extrudeuse et un séchage consécutif sous pression.

7. Procédé selon la revendication 6, **caractérisé en ce qu'**il comprend les étapes suivantes :
(a) mettre en suspension et, le cas échéant, comprimer la cellulose microcristalline ou pulvérisée humide ou non séchée, non cornée dans un milieu aqueux pour obtenir une dispersion homogène ;
(b) mélanger la suspension obtenue à l'étape (a) ou des masses pâteuses préalablement comprimées après refroidissement en dessous de 40 °C avec la polyvinylpyrrolidone réticulée ;
(c) procéder le cas échéant à un séchage préalable;
(d) procéder à un passage par compression, de préférence, à un pressage de la masse plastique humide obtenue à l'étape (b) ou (c) et préalablement comprimée à travers un crible, un disque perforé ou une extrudeuse ;
(e) sécher la masse agrégée obtenue à l'étape (d) à une température de 20 à 90 °C ; et
(f) broyer le produit ainsi obtenu selon une granulométrie moyenne de 10 à 2000 µm, de préférence, de 10 à 1000 µm.

8. Procédé selon la revendication 7, **caractérisé en ce qu'**on effectue le mélange et la compression par malaxage.

9. Procédé selon la revendication 6, **caractérisé en ce qu'**il comprend les étapes suivantes :
(a) mélanger les composants pulvérulents de la polyvinylpyrrolidone réticulée et de la cellulose ;
(b) procéder à un criblage à travers un crible fin ;
(c) procéder à une compression par compactage ou par briquetage à l'aide de cylindres de compactage ou d'une presse excentrique à des pressions de 20 à 60 kN, de préférence, de 30 à 50 kN ;
(d) procéder à une granulation ou à une homogénéisation réductrice de la briquettes ou de la plaquette obtenue selon l'étape (c) avec des cylindres dentés ou un appareil analogue ou bien à un broyage avec un broyeur adapté selon une granulométrie de 10 à 2000 µm ; et
(e) procéder au broyage du produit ainsi obtenu selon une granulométrie moyenne de 10 à 1000 µm.

10. Utilisation du produit polysaccharidique selon les revendications 1 à 5 en tant que stabilisant pour dispersion ou pour suspension lors de l'élaboration de préparations fluides et semi-solides.

11. Utilisation du produit polysaccharidique selon les revendications 1 à 5 en tant que matière explosive sous forme de pastilles.
